# EUROPÄISCHE PATENTSCHRIFT

(11) **EP 2 863 831 B1**
(45) Veröffentlichungstag und Bekanntmachung des Hinweises auf die Patenterteilung: **07.09.2016**
(21) Anmeldenummer: 13735224.1
(22) Anmeldetag: 25.06.2013
(51) Int. Cl.: A61C 9/00, A61C 7/00, A61B 5/055, A61B 6/14, A61B 5/00, A61B 6/03

(54) **VERFAHREN ZUR ÜBERPRÜFUNG VON ZAHNSTELLUNGEN**
METHOD FOR CHECKING TOOTH POSITIONS
PROCÉDÉ DE CONTRÔLE DE POSITIONS DENTAIRES

(30) Priorität: 25.06.2012 DE 102012210758
(43) Veröffentlichungstag der Anmeldung: 29.04.2015
(73) Patentinhaber: Sirona Dental Systems GmbH, 64625 Bensheim (DE)
(72) Erfinder: WUNDRAK, Stefan, 64625 Gronau (DE); LINDENBERG, Kai, 64395 Wersau (DE)
(74) Vertreter: Sommer, Peter
(86) Internationale Anmeldenummer: PCT/EP2013/063196
(87) Internationale Veröffentlichungsnummer: WO 2014/001284

(56) Entgegenhaltungen:
- US-A1- 2004 197 727
- US-A1- 2009 325 127
- US-A1- 2011 268 327

## Beschreibung

### Technisches Gebiet

Die Erfindung betrifft ein Verfahren zur Überprüfung von Zahnstellungen, wobei eine initiale dreidimensionale Volumenaufnahme von zu überprüfenden Zähnen durchgeführt wird, wobei anhand der initialen Volumenaufnahme die Lage und die Orientierung der zu überprüfenden Zähne bestimmt wird. Die zu überprüfenden Zähne sind dabei natürliche Zähne bestehend aus Zahnstümpfen und Zahnwurzeln und/oder künstliche Zähne bestehend aus künstlichen Zahnstümpfen und Implantaten, wobei insbesondere die Lagebeziehung und Orientierung der Zahnstümpfe relativ zu den Zahnwurzeln und/oder zu den Implantaten bestimmt werden.

### Stand der Technik

Aus dem Stand der Technik sind mehrere Verfahren zur zeitlichen Verfolgung von Zahnstellungen bekannt, wobei meist dreidimensionale Röntgenaufnahmeverfahren, wie CT-Aufnahmeverfahren, verwendet werden, z.B. US 2011/0268327. Anhand der erzeugten Röntgenaufnahmen werden dann die Zahnstellungen überprüft.

Der Nachteil dieser Verfahren besteht darin, dass aufgrund der Dosisbelastung durch die Röntgenstrahlung eine engmaschige Beobachtung der Zahnstellungen oft nicht zu rechtfertigen ist.

Die Aufgabe der vorliegenden Erfindung besteht daher darin ein Verfahren zur Überprüfung von Zahnstellungen bereitzustellen, das eine engmaschige Beobachtung mit einer geringen Dosisbelastung ermöglicht.

### Darstellung der Erfindung

Die Erfindung betrifft ein Verfahren zur Überprüfung von Zahnstellungen, wobei eine initiale dreidimensionale Volumenaufnahme von zu überprüfenden Zähnen durchgeführt wird, wobei anhand der initialen Volumenaufnahme die Lage und die Orientierung der zu überprüfenden Zähne bestimmt wird. Die zu überprüfenden Zähne sind dabei natürliche Zähne bestehend aus Zahnstümpfen und Zahnwurzeln und/oder künstliche Zähne bestehend aus künstlichen Zahnstümpfen und Implantaten. Insbesondere die Lagebeziehung und Orientierung der Zahnstümpfe relativ zu den Zahnwurzeln und/oder zu den Implantaten werden dabei bestimmt. Anschließend wird zur Überprüfung der Zahnstellungen über einen längeren Zeitraum in regelmäßigen zeitlichen Abständen mindestens eine erste optische Oberflächenkontrollaufnahme der Zähne durchgeführt, wobei unter Verwendung der bestimmten Lagebeziehung ausgehend von der Lage der Oberflächen der Zahnstümpfe aus der optischen Oberflächenkontrollaufnahme die Lage und Orientierung der Zahnwurzeln und/oder der Implantate relativ zueinander und/oder relativ zu einem Kieferknochen bestimmt werden.

Die Bestimmung der Lagebeziehung erfolgt dabei computerstützt automatisch, wobei die Zahnstümpfe, die Zahnwurzeln und/oder die Implantate unter Verwendung eines Segmentierungsverfahrens automatisch registriert werden und anschließend die Lagebeziehung computergestützt automatisch bestimmt wird. Anschließend werden 3D-Modelle der segmentierten Zähne solange gedreht und/oder verschoben, bis die Oberflächen der Zahnstumpfe dieser 3D-Modelle mit den Oberflächen der Zahnstümpfe aus der optischen Oberflächenkontrollaufnahme in Überlagerung gebracht werden.

Die grundlegend Idee des vorliegenden Verfahrens liegt in der Annahme, dass die Form der einzelnen Zähne unverändert bleibt und lediglich die Lage und Orientierung der Zähne und Ihrer Zahnwurzeln relativ zum Kieferknochen verändert wird. Folglich kann aus der Lage der Oberflächen der Zahnstümpfe aus der optischen Oberflächenkontrolleaufnahme die Lage und Orientierung der Zahnwurzel berechnet werden.

Die Überprüfung von Zahnstellungen über einen längeren Zeitraum ist vor allem bei Zahnkorrekturen mittels Zahnspangen erforderlich, die beispielsweise Fehlbiss, Kreuzbiss, Vorbiss, Fehlwuchs der Zähne oder des Kiefers, korrigieren. Die initiale dreidimensionale Volumenaufnahme kann beispielsweise mittels eines CT-Röntgengeräts oder einer MRT-Vorrichtung durchgeführt werden. Die erzeugte initiale Volumenaufnahme kann dann anschließend mittels eines Segmentierungsverfahrens analysiert werden und die Zähne, umfassend Zahnstümpfe und Zahnwurzeln bzw. Implantate, können segmentiert und anschließend registriert werden. Die dreidimensionale optische Oberflächenkontrollaufnahme kann beispielsweise mittels eines Streifenprojektionsverfahrens unter Verwendung einer dentalen Kamera oder auch mittels eines digitalisierten Abdrucks durchgeführt werden. Ein digitalisierter Abdruck ist dabei eine dreidimensionale Aufnahme eines plastischen Zahn-/Gebissabdrucks des dentalen Objekts, wobei die Vermessung unter Verwendung des Streifenprojektionsverfahrens, durch CT-Vermessung, durch MRT-Vermessung oder durch mechanische Abtastung erfolgen kann.

Bei der Berechnung der Lage der Zahnwurzeln und/oder der Implantate können beispielsweise 3D-Modelle der segmentierten Zähne aus der initialen Volumenaufnahme verwendet werden, wobei diese 3D-Modelle solange gedreht und verschoben werden, bis die Oberflächen der Zahnstümpfe dieser 3D-Modelle zu den Oberflächen der Zahnstümpfe aus den optischen Oberflächenkontrollaufnahmen passen. Dieses Anpassungsverfahren der 3D-Modelle kann auch computergestützt automatisch erfolgen, wobei die Oberflächen der Zahnstümpfe der 3D-Modelle mit den Oberflächen der Zahnstümpfe aus den optischen Oberflächenkontrollaufnahmen in Überlagerung gebracht werden.

Dadurch wird auf einfache Art und Weise eine sichere Ermittlung der Lage und Orientierung der Zahnwurzel gewährleistet.

Die Zahnstümpfe können verschiedene Teile aus Hartgewebe sein, die aus dem Zahnfleisch herausragen und bei einer optischen Aufnahme erfasst werden können. Zahnstümpfe können beispielsweise vollständige Zahnkronen oder auch nur Teile von natürlichen oder künstlichen Zähnen, wie Präparationen oder Abutments, sein, die aus dem Zahnfleisch herausragen.

Bei dem Segmentierungsverfahren wird die initiale Volumenaufnahme und die darauffolgenden optischen Oberflächenkontrollaufnahmen automatisch nach den Mustern der Zahnstümpfe, der Zahnwurzeln und/oder der Implantate durchsucht und diese vom umgebenden Gewebe segmentiert.

Ein Vorteil des vorliegenden Verfahrens liegt darin, dass der Patient nur während der initialen Volumenaufnahme, wie einer Röntgenaufnahme, einer Dosisbelastung ausgesetzt wird, wobei anschließend für die weitere Überprüfung der Zahnstellungen lediglich dreidimensionale optische Oberflächenkontrollaufnahmen der Zahnsituation durchgeführt werden. Auf diese Weise wird eine engmaschige Beobachtung mit einer geringen Dosisbelastung ermöglicht.

Vorteilhafterweise kann anhand der initialen dreidimensionalen Volumenaufnahme die Oberflächen der Zahnstümpfe und die Lage der Zahnwurzeln und/oder der Implantate bestimmt werden, wobei die Lagebeziehung allein anhand der Volumenaufnahme bestimmt wird.

Dadurch werden sowohl die Zahnstümpfe als auch die Zahnwurzeln und/oder die Implantate in der Volumenaufnahme, beispielsweise mittels eines Segmentierungsverfahrens, erkannt, wobei anschließend die Lagebeziehung bestimmt wird.

Vorteilhafterweise kann zur Bestimmung der Lagebeziehung zusätzlich zur initialen Volumenaufnahme eine initiale optische Oberflächenaufnahme der zu überprüfenden Zähne durchgeführt werden, wobei die Lagebeziehung zwischen der Lage der Zahnwurzeln und/oder der Implantate aus der initialen Volumenaufnahme und der Lage der Oberflächen der Zahnstümpfe aus der initialen optischen Oberflächenaufnahme bestimmt wird.

Bei dieser Alternative wird die Lagebeziehung zwischen den Zahnstümpfen und den Zahnwurzeln und/oder den Implantaten durch Vergleich der initialen Volumenaufnahme mit der initiale optische Oberflächenaufnahme bestimmt. Auf diese Art und Weise wird eine einfache und fehlerfreie Bestimmung der Lagebeziehung ermöglicht.

Vorteilhafterweise kann die initiale Volumenaufnahme und die initiale optische Oberflächenaufnahme während einer ersten Untersuchung innerhalb einer Zeitspanne von maximal 4 Stunden durchgeführt werden.

Dadurch wird also die Lagebeziehung bei einem ersten Untersuchungstermin bestimmt. Anschließend wird in regelmäßigen zeitlichen Abständen bei Kontrollterminen die Lage der Zahnwurzeln und/oder der Implantate anhand der Oberflächekontrollaufnahmen überprüft.

Vorteilhafterweise kann die Bestimmung der Lagebeziehung computerstützt manuell erfolgen, wobei die Zahnstümpfe, die Zahnwurzeln und/oder die Implantate manuell durch einen Benutzer unter Verwendung von Eingabemitteln ausgewählt werden.

Bei dieser Alternative erfolgt die Segmentierung manuell durch den Benutzer, wobei der Benutzer die Zahnstümpfe oder die Zahnwurzeln virtuell über Eingabemittel mittels eines Cursors umrandet und damit die Abmessung sowie die Lage und Ausrichtung relativ zueinander festlegt.

Vorteilhafterweise kann die initiale dreidimensionale Volumenaufnahme eine dreidimensionale CT-Röntgenaufnahme sein.

Dadurch wird insbesondere hartes Gewebe, wie Zahngewebe und Kieferknochen, besonders deutlich abgebildet.

Vorteilhafterweise kann die initiale dreidimensionale Volumenaufnahme eine dreidimensionale MRT-Aufnahme sein.

Dadurch wird weiches Gewebe, wie Zahnfleisch, besonders deutlich abgebildet.

Vorteilhafterweise kann die optische Oberflächenkontrollaufnahme mittels einer optischen Vermessungsvorrichtung unter Verwendung eines Streifenprojektionsverfahrens durchgeführt werden.

Die optische Vermessungsvorrichtung kann beispielsweise ein dentales Handstück sein, das die Zahnsituation unter Verwendung des Streifenprojektionsverfahrens vermisst und dabei eine dreidimensionale optische Aufnahme erstellt.

Vorteilhafterweise kann die optische Oberflächenkontrollaufnahme unter Verwendung eines digitalisierten Abdrucks der Zähne erzeugt werden.

Vorteilhafterweise können zur Überprüfung weitere optische Oberflächenkontrollaufnahmen der zu überprüfenden Zähne durchgeführt werden. Anschließend können ausgehend von der Lage der Oberflächen der Zahnstümpfe aus den optischen Oberflächenkontrollaufnahmen die Lage und Orientierung der Zahnwurzeln und/oder der Implantate relativ zueinander und/oder relativ zu einem Kieferknochen bestimmt werden.

Vorteilhafterweise können die anhand der Oberflächen der Zahnstümpfe aus der optischen Oberflächenkontrollaufnahme bestimmten Zahnwurzeln und/oder Implantate graphisch mittels einer Anzeigevorrichtung relativ zueinander und/oder in Relation zum Kieferknochen aus der initialen Volumenaufnahme dargestellt werden.

Die Anzeigevorrichtung kann beispielsweise ein Monitor sein, der die initiale Röntgenaufnahme in Überlagerung mit den berechneten 3D-Modellen der Zähne darstellt. Dadurch wird es dem Benutzer ermöglicht, die Orientierung der Zahnwurzeln in Relation zum Kieferknochen besser einzuschätzen.

Vorteilhafterweise können die Oberflächen der registrierten Zahnstümpfe aus der initialen optischen Aufnahme in einem Datenspeicher abgespeichert werden. Die weiteren optischen Aufnahmen können dann mittels eines Mustererkennungsalgorithmus automatisch nach diesen registrierten Oberflächen der Zahnstümpfe durchsucht werden, wobei anschließend die Lage und die Orientierung der Zahnstümpfen relativ zueinander und/oder relativ zum Kieferknochen automatisch computergestützt bestimmt werden.

Durch die Verwendung des Mustererkennungsalgorithmus wird die Suche nach den Oberflächen der Zahnstümpfe in den optischen Aufnahmen vereinfacht.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens;
- Fig. 2: eine Skizze zur Verdeutlichung des vorliegenden Verfahrens, wobei ein Zahn im Vergleich zu Fig. 1 verdreht und verschoben ist.

### Ausführungsbeispiel

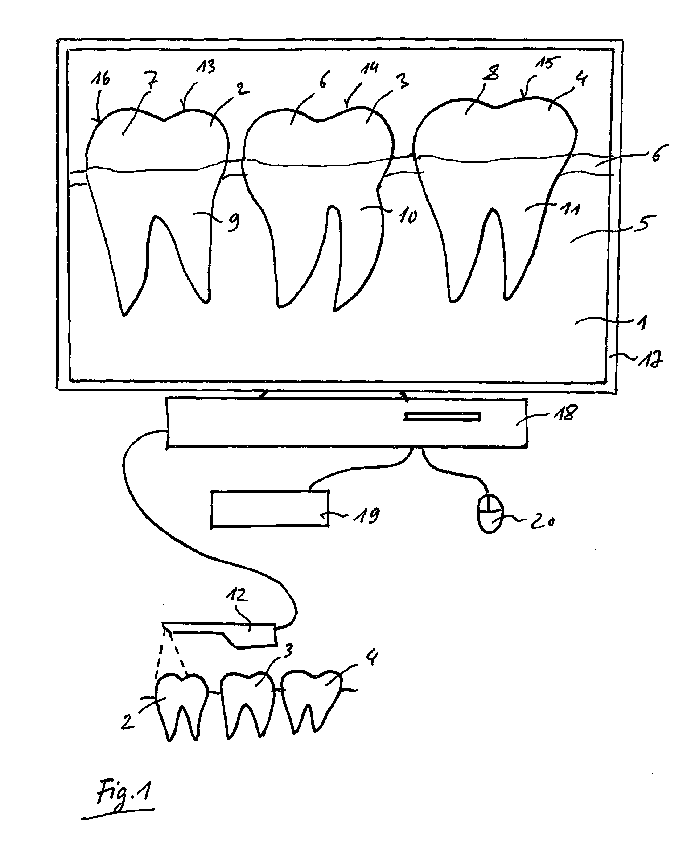

Die Fig. 1 zeigt eine Skizze zur Verdeutlichung des vorliegenden Verfahrens zur Überprüfung von Zahnstellungen. Im ersten Verfahrensschritt wird eine initiale dreidimensionale Röntgenaufnahme 1 mittels eines Röntgensystems wie eines CT-Röntgensystems, durchgeführt. Die Röntgenaufnahme umfasst die zu überprüfenden Zähne 2, 3 und 4 sowie den Kieferknochen 5 und eine Gingivaschicht 6. Anhand der initialen Röntgenaufnahme 5 werden dann die Lage und die Orientierung der zu überprüfenden Zähne 2, 3, 4 insbesondere von Zahnstümpfe 7, 6 und 8 und/oder der Zahnwurzeln 9, 10 und 11 registriert. Im nächsten Verfahrensschritt wird zusätzlich zur initialen Röntgenaufnahme eine initiale optische Aufnahme der zu überprüfenden Zähne 2, 3 und 4 mittels der Vermessungsvorrichtung 12, die auf einem Streifenprojektionsverfahren beruhen kann, durchgeführt. Im weiteren Verfahrensschritt wird eine Lagebeziehung zwischen der Lage der zu überprüfenden Zähne 2, 3 und 4 in der initialen Röntgenaufnahme 5 und den Oberflächen 13, 14 und 15 der Zahnstümpfe 7, 6 und 8 aus der initialen optischen Aufnahme bestimmt. Die initiale optische Aufnahme 16, die die Zahnsituation und insbesondere die Oberflächen 13, 14 und 15 der Zahnstümpfe 7, 6 und 8 umfasst wird mittels einer Anzeigevorrichtung 17, wie mittels eines Monitors, in Überlagerung mit der dreidimensionalen Röntgenaufnahme 5 dargestellt. Die Lagebeziehung zwischen den beiden Aufnahmen 5 und 16 wird dadurch bestimmt, dass übereinstimmende Strukturen, wie die Oberflächen 13, 14 und 15 der Zahnstümpfe 7, 6 und 8 automatisch computergestützt mittels des Computers 18 in Übereinstimmung gebracht werden. Das Bestimmen der Lagebeziehung kann alternativ auch manuell durch einen Benutzer unter Verwendung der Eingabemittel, wie einer Tastatur 19 und einer Maus 20, erfolgen. Im weiteren Verfahrensschritt wird unter Verwendung der bestimmten Lagebeziehung ausgehend von der Lage der Oberflächen 13, 14 und 15 der Zahnstümpfe 7, 6 und 8 aus der optischen Aufnahme 16 die Lage und Orientierung der Zahnwurzeln 9, 10 und 11 zueinander und/oder relativ zum Kieferknochen 5 berechnet. Anschließend werden in regelmäßigen zeitlichen Abständen weitere optische dreidimensionale Aufnahmen der zu überprüfenden Zähne 2, 3 und 4 mittels der Vermessungsvorrichtung 12 durchgeführt. Ausgehend von der Lage der Oberflächen 13, 14 und 15 wird dann die veränderte Lage und Orientierung der Zahnwurzeln 9, 10 und 11 relativ zueinander und/oder relativ zum Kieferknochen 5 berechnet. Die Registrierung der Zahnstümpfe 6, 7 und 8 in der initialen Röntgenaufnahme und in der initialen optischen Aufnahme sowie die Registrierung der Zahnwurzeln 9, 10 und 11 in der initialen Röntgenaufnahme können mittels eines Segmentierungsverfahrens erfolgen, dass die Aufnahmen mittels des Computers 18 analysiert und die Zähne 2, 3 und 4 segmentiert. Die segmentierten Zähne 2, 3 und 4 können auch als 3D-Modelle in einem Datenspeicher abgespeichert werden, wobei die weiteren optischen Aufnahmen mittels eines Mustererkennungsalgorithmus nach diesen 3D-Modellen der Zähne durchsucht werden können.

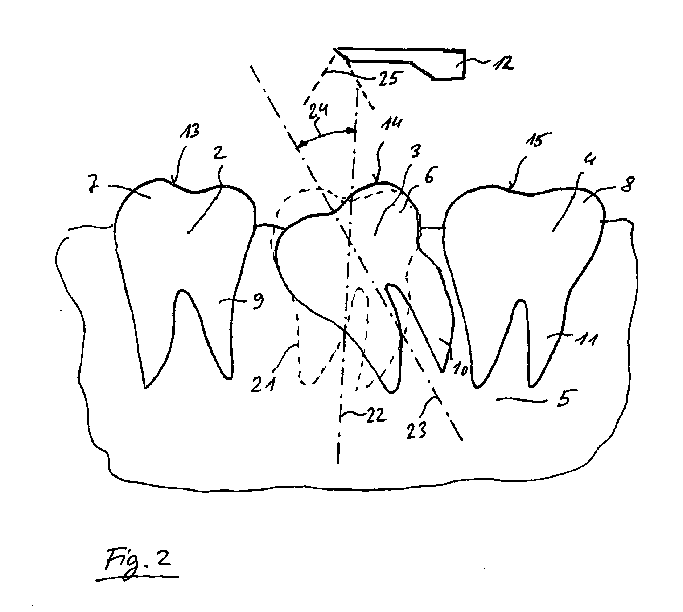

Die Fig. 2 zeigt eine Skizze zur Verdeutlichung des vorliegenden Verfahrens, wobei im Vergleich zu Fig. 1 der mittlere Zahn 3 sich relativ zu den Nachbarzähnen 2 und 4 sowie relativ zum Kieferknochen 5 gedreht und verschoben hat. Die ursprüngliche Lage und Orientierung des Zahns 3 ist durch die gestrichelte Linie 21 dargestellt. Eine Zahnmittelachse des mittleren Zahns 3 hat sich somit von einer ursprünglichen Position 22 zu einer neuen Position 23 um einen Winkel 24 verschoben. Zur Überprüfung der Zahnstellungen wird eine optische Oberflächenkontrollaufnahme 25 mittels der Vermessungsvorrichtung 12 aus Fig. 1 durchgeführt, wobei die Oberflächen 13, 14 und 15 der Zahnstümpfe 7, 6 und 8 erneut erfasst werden. Anschließend wird ausgehend von der Lage der Oberflächen 13, 14 und 15 die Lage und Orientierung der Zahnwurzeln 9, 10 und 11 relativ zueinander und/oder relativ zum Kieferknochen 5 berechnet. Auf diese Weise können damit in kurzen zeitlichen Abständen mehrere Oberflächenkontrollaufnahmen durchgeführt werden, um eine genaue Überprüfung der Zahnstellungen mit einer nur geringen Dosisbelastung zu ermöglichen.

### Bezugszeichen

- 1: initiale Röntgenaufnahme
- 2: erster Zahn
- 3: zweiter Zahn
- 4: dritter Zahn
- 5: Kieferknochen
- 6: Zahnkrone
- 7: Zahnkrone
- 8: Zahnkrone
- 9: Zahnwurzel
- 10: Zahnwurzel
- 11: Zahnwurzel
- 12: Vermessungsvorrichtung
- 13: erste Oberfläche der Zahnkrone
- 14: zweite Oberfläche der Zahnkrone
- 15: dritte Oberfläche der Zahnkrone
- 16: initiale optische Aufnahme
- 17: Anzeigevorrichtung, Monitor
- 18: Computer
- 19: Tastatur
- 20: Maus
- 21: gestrichelte Linie
- 22: ursprüngliche Position
- 23: neue Position
- 24: Winkel
- 25: optische Oberflächenkontrollaufnahme

## Patentansprüche

1. Verfahren zur Überprüfung von Zahnstellungen, wobei eine initiale dreidimensionale Volumenaufnahme (1) von zu überprüfenden Zähnen (2, 3, 4) durchgeführt wird, wobei anhand der initialen Volumenaufnahme (1) die Lage und die Orientierung der zu überprüfenden Zähne (2, 3, 4) bestimmt wird, wobei die zu überprüfenden Zähne natürliche Zähne (2, 3, 4) bestehend aus Zahnstümpfen (6, 7, 8) und Zahnwurzeln (9, 10, 11) und/oder künstliche Zähne bestehend aus künstlichen Zahnstümpfen und Implantaten sind, wobei insbesondere die Lagebeziehung und Orientierung der Zahnstümpfe (6, 7, 8) relativ zu den Zahnwurzeln (9, 10, 11) und/oder zu den Implantaten bestimmt werden, **dadurch gekennzeichnet, dass** zur Überprüfung der Zahnstellungen über einen längeren Zeitraum in regelmäßigen zeitlichen Abständen mindestens eine erste optische Oberflächenkontrollaufnahme (25) der Zähne (2, 3, 4) durchgeführt wird, wobei unter Verwendung der bestimmten Lagebeziehung ausgehend von der Lage der Oberflächen (13, 14, 15) der Zahnstümpfe (6, 7, 8) aus der optischen Oberflächenkontrollaufnahme (25) die Lage und Orientierung der Zahnwurzeln (9, 10, 11) und/oder der Implantate relativ zueinander und/oder relativ zu einem Kieferknochen (5) bestimmt werden, wobei die Bestimmung der Lagebeziehung computerstützt automatisch erfolgt, wobei die Zähne (2, 3, 4) umfassend die Zahnstümpfe (6, 7, 8), die Zahnwurzeln (9, 10, 11) und/oder die Implantate durch die Analyse der initialen Volumenaufnahme (1) unter Verwendung eines Segmentierungsverfahrens automatisch registriert werden und anschließend die Lagebeziehung computergestützt automatisch bestimmt wird, wobei 3D-Modelle der segmentierten Zähne (2, 3, 4) solange gedreht und/oder verschoben werden, bis die Oberflächen (13, 14, 15) der Zahnstümpfe (6, 7, 8) dieser 3D-Modelle mit den Oberflächen der Zahnstümpfe (6, 7, 8) aus der optischen Oberflächenkontrollaufnahme (25) in Überlagerung gebracht werden.

2. Verfahren nach Anspruch 1, **dadurch gekennzeichnet, dass** anhand der initialen dreidimensionalen Volumenaufnahme (1) die Oberflächen (13, 14, 15) der Zahnstümpfe (6, 7, 8) und die Lage der Zahnwurzeln und/oder der Implantate bestimmt werden, wobei die Lagebeziehung allein anhand der Volumenaufnahme (1) bestimmt wird.

3. Verfahren nach Anspruch 1, **dadurch gekennzeichnet, dass** zur Bestimmung der Lagebeziehung zusätzlich zur initialen Volumenaufnahme (1) eine initiale optische Oberflächenaufnahme (16) der zu überprüfenden Zähne (2, 3, 4) durchgeführt wird, wobei die Lagebeziehung zwischen der Lage der Zahnwurzeln (9, 10, 11) und/oder der Implantate (2, 3, 4) aus der initialen Volumenaufnahme (1) und den Oberflächen (13, 14, 15) der Zahnstümpfe (6, 7, 8) aus der initialen optischen Oberflächenaufnahme (16) bestimmt wird.

4. Verfahren nach Anspruch 3, **dadurch gekennzeichnet, dass** die initiale Volumenaufnahme (1) und die initiale optische Oberflächenaufnahme (16) während einer ersten Untersuchung innerhalb einer Zeitspanne von maximal 4 Stunden durchgeführt werden.

5. Verfahren nach einem der Ansprüche 1 bis 4, **dadurch gekennzeichnet, dass** die initiale dreidimensionale Volumenaufnahme eine dreidimensionale CT-Röntgenaufnahme (1) ist.

6. Verfahren nach einem der Ansprüche 1 bis 4, **dadurch gekennzeichnet, dass** die initiale dreidimensionale Volumenaufnahme eine dreidimensionale MRT-Aufnahme ist.

7. Verfahren nach einem der Ansprüche 1 bis 6, **dadurch gekennzeichnet, dass** die optische Oberflächenkontrollaufnahme (25) mittels einer optischen Vermessungsvorrichtung (12) unter Verwendung eines Streifenprojektionsverfahrens durchgeführt wird.

8. Verfahren nach einem der Ansprüche 1 bis 6, **dadurch gekennzeichnet, dass** die optische Oberflächenkontrollaufnahme (25) unter Verwendung eines digitalisierten Abdrucks der Zähne (2, 3, 4) erzeugt wird.

9. Verfahren nach einem der Ansprüche 1 bis 8, **dadurch gekennzeichnet, dass** zur Überprüfung weitere optische Oberflächenkontrollaufnahmen (25) der zu überprüfenden Zähne (2, 3, 4) durchgeführt werden und anschließend ausgehend von der Lage der Oberflächen (13, 14, 15) der Zahnstümpfe (6, 7, 8) aus den optischen Oberflächenkontrollaufnahmen (25) die Lage und Orientierung der Zahnwurzeln (9, 10, 11) und/oder der Implantate relativ zueinander und/oder relativ zu einem Kieferknochen (5) bestimmt werden.

10. Verfahren nach einem der Ansprüche 1 bis 9, **dadurch gekennzeichnet, dass** die anhand der Oberflächen (13, 14, 15) der Zahnstümpfe (6, 7, 8) aus der optischen Oberflächenkontrollaufnahme (25) bestimmten Zahnwurzeln (9, 10, 11) und/oder Implantate graphisch mittels einer Anzeigevorrichtung (17) relativ zueinander und/oder in Relation zum Kieferknochen (5) aus der initialen Volumenaufnahme (1) dargestellt werden.

11. Verfahren nach einem der Ansprüche 1 bis 10, **dadurch gekennzeichnet, dass** die Oberflächen (13, 14, 15) der registrierten Zahnstümpfe (6, 7, 8) aus der initialen dreidimensionalen Volumenaufnahme (1) und/oder der initialen optischen Oberflächenaufnahme (16) in einem Datenspeicher abgespeichert werden und die optischen Oberflächenkontrollaufnahmen (25) mittels eines Mustererkennungsalgorithmus automatisch nach diesen registrierten Oberflächen (13, 14, 15) der Zahnstümpfe (6, 7, 8) durchsucht werden, wobei anschließend die Lage und die Orientierung der Zahnstümpfe (6, 7, 8) relativ zueinander und/oder relativ zum Kieferknochen (5) automatisch computergestützt bestimmt werden.

## Claims

1. A method for checking tooth positions, wherein an initial three-dimensional volumetric image (1) of teeth (2, 3, 4) to be checked is made, wherein by using the initial volumetric image (1), the position and orientation of the teeth (2, 3, 4) to be checked are determined, wherein the teeth to be checked are natural teeth (2, 3, 4) consisting of tooth stumps (6, 7, 8) and tooth roots (9, 10, 11), and/or artificial teeth consisting of artificial tooth stumps and implants, wherein in particular the positional relationship and orientation of the tooth stumps (6, 7, 8) relative to the tooth roots (9, 10, 11) and/or to the implants are determined, **characterized in that** in order to check the tooth positions over a longer period of time at regular intervals, at least one first optical surface control image (25) of the teeth (2, 3, 4) is taken, wherein the position and orientation of the tooth roots (9, 10, 11) and/or the implants relative to each other, and/or relative to a jaw bone (5) are determined using the determined positional relationship based on the position of the surfaces (13, 14, 15) of the tooth stumps (6, 7, 8) from the optical surface control image (25), wherein the positional relationship is determined automatically by computer, wherein the teeth (2, 3, 4) comprising the tooth stumps (6, 7, 8), the tooth roots (9, 10, 11) and/or the implants are automatically registered by analyzing the initial volumetric image (1) using a segmentation method, and then the positional relationship is determined automatically by computer, wherein 3-D models of the segmented teeth (2, 3, 4) are rotated and/or shifted until the surfaces (13, 14, 15) of the tooth stumps (6, 7, 8) of these 3-D models are caused to overlap with the surfaces of the tooth stumps (6, 7, 8) from the optical surface control image (25).

2. The method according to claim 1, **characterized in that** by using the initial three-dimensional volumetric image (1), the surfaces (13, 14, 15) of the tooth stumps (6, 7, 8) and the position of the tooth roots and/or implants are determined, wherein the positional relationship is determined using only the volumetric image (1).

3. The method according to claim 1, **characterized in that** in order to determine the positional relationship, an initial optical surface image (16) of the teeth (2, 3, 4) to be checked is made in addition to the initial volumetric image (1), wherein the positional relationship between the position of the tooth roots (9, 10, 11) and/or the implants (2, 3, 4) is determined from the initial volumetric image (1) and the surfaces (13, 14, 15) of the tooth stumps (6, 7, 8) from the initial optical surface image (16).

4. The method according to claim 3, **characterized in that** the initial volumetric image (1) and the initial optical surface image (16) are taken during an initial examination within a time period of a maximum of 4 hours.

5. The method according to one of claims 1 to 4, **characterized in that** the initial three-dimensional volumetric image is a three-dimensional CT x-ray image (1).

6. The method according to one of claims 1 to 4, **characterized in that** the initial three-dimensional volumetric image is a three-dimensional MRT image.

7. A method according to one of claims 1 to 6, **characterized in that** the optical surface control image (25) is taken with an optical measuring device (12) using a fringe projection method.

8. The method according to one of claims 1 to 6, **characterized in that** the optical surface control image (25) is generated using a digitized impression of the teeth (2, 3, 4).

9. The method according to one of claims 1 to 8, **characterized in that** for checking, additional optical surface control images (25) of the teeth (2, 3, 4) to be checked are taken, and subsequently the position and orientation of the tooth roots (9, 10, 11) and/or of the implants relative to each other and/or relative to a jaw bone (5) are determined based on the position of the surfaces (13, 14, 15) of the tooth stumps (6, 7, 8) from the optical surface control images (25).

10. The method according to one of claims 1 to 9, **characterized in that** the tooth roots (9, 10, 11) and/or implants determined with reference to the surfaces (13, 14, 15) of the tooth stumps (6, 7, 8) from the optical surface control image (25) are graphically depicted by means of a display device (17) relative to each other, and/or in relation to the jaw bone (5) from the initial volumetric image (1).

11. The method according to one of claims 1 to 10, **characterized in that** the surfaces (13, 14, 15) of the registered tooth stumps (6, 7, 8) from the initial three-dimensional volumetric image (1), and/or the initial optical surface image (16) are saved in a data memory, and the optical surface control images (25) are automatically searched by means of a pattern recognition algorithm for these registered surfaces (13, 14, 15) of the tooth stumps (6, 7, 8), wherein the position and orientation of the tooth stumps (6, 7, 8) relative to each other and relative to the jaw bone (5) are then automatically determined by computer.

## Revendications

1. Procédé de contrôle de positions dentaires, un cliché volumique (1) initial en trois dimensions de dents à contrôler (2, 3, 4) étant pris, la position et l'orientation des dents à contrôler (2, 3, 4) étant déterminées à l'aide du cliché volumique (1) initial, les dents à contrôler étant des dents naturelles (2, 3, 4) constituées de moignons dentaires (6, 7, 8) et de racines dentaires (9, 10, 11) et/ou des dents artificielles constituées de moignons dentaires artificiels et d'implants, la position relative et l'orientation des moignons dentaires (6, 7, 8) par rapport aux racines dentaires (9, 10, 11) et/ou par rapport aux implants étant notamment déterminées, **caractérisé en ce que**, pour contrôler les positions dentaires pendant une période de temps prolongée à intervalles temporels réguliers, au moins un premier cliché optique de contrôle de surface (25) des dents (2, 3, 4) est pris, la position et l'orientation des racines dentaires (9, 10, 11) et/ou des implants les uns par rapport aux autres et/ou par rapport à un os maxillaire (5) étant déterminées à partir de la position des surfaces (13, 14, 15) des moignons dentaires (6, 7, 8) du cliché optique de contrôle de surface (25) en utilisant la position relative déterminée, la détermination de la position relative se faisant de manière automatique assistée par ordinateur, les dents (2, 3, 4) avec les moignons dentaires (6, 7, 8), les racines dentaires (9, 10, 11) et/ou les implants étant enregistrées automatiquement par l'analyse du cliché volumique (1) initial en utilisant un procédé de segmentation et la position relative étant ensuite déterminée de manière automatique assistée par ordinateur, des modèles 3D des dents (2, 3, 4) segmentées étant tournés et/ou glissés jusqu'à ce que les surfaces (13, 14, 15) des moignons dentaires (6, 7, 8) de ces modèles 3D soient amenées en superposition avec les surfaces des moignons dentaires (6, 7, 8) du cliché optique de contrôle de surface (25).

2. Procédé selon la revendication 1, **caractérisé en ce que**, à l'aide du cliché volumique (1) initial en trois dimensions, les surfaces (13, 14, 15) des moignons dentaires (6, 7, 8) et la position des racines dentaires et/ou des implants sont déterminées, la position relative étant déterminée uniquement à l'aide du cliché volumique (1).

3. Procédé selon la revendication 1, **caractérisé en ce que**, pour déterminer la position relative, un cliché optique initial de surface (16) des dents à contrôler (2, 3, 4) est pris en plus du cliché volumique (1) initial, la position relative entre la position des racines dentaires (9, 10, 11) et/ou des implants (2, 3, 4) du cliché volumique (1) initial et les surfaces (13, 14, 15) des moignons dentaires (6, 7, 8) du cliché optique initial de surface (16) étant déterminée.

4. Procédé selon la revendication 3, **caractérisé en ce que** le cliché volumique (1) initial et le cliché optique initial de surface (16) sont pris pendant un premier examen dans un laps de temps de 4 heures maximum.

5. Procédé selon l'une des revendications 1 à 4, **caractérisé en ce que** le cliché volumique initial en trois dimensions est une radiographie CT (1) en trois dimensions.

6. Procédé selon l'une des revendications 1 à 4, **caractérisé en ce que** le cliché volumique initial en trois dimensions est une image IRM en trois dimensions.

7. Procédé selon l'une des revendications 1 à 6, **caractérisé en ce que** le cliché optique de contrôle de surface (25) est pris au moyen d'un dispositif de mesure optique (12) en utilisant un procédé de projection de franges.

8. Procédé selon l'une des revendications 1 à 6, **caractérisé en ce que** le cliché optique de contrôle de surface (25) est produit en utilisant une empreinte numérisée des dents (2, 3, 4).

9. Procédé selon l'une des revendications 1 à 8, **caractérisé en ce que**, pour le contrôle, d'autres clichés optiques de contrôle de surface (25) des dents à contrôler (2, 3, 4) sont pris et la position et l'orientation des racines dentaires (9, 10, 11) et/ou des implants les uns par rapport aux autres et/ou par rapport à l'os maxillaire (5) sont ensuite déterminées à partir de la position des surfaces (13, 14, 15) des moignons dentaires (6, 7, 8) sur les clichés de contrôle de surface (25).

10. Procédé selon l'une des revendications 1 à 9, **caractérisé en ce que** les racines dentaires (9, 10, 11) et/ou implants déterminés à l'aide des surfaces (13, 14, 15) des moignons dentaires (6, 7, 8) sur le cliché optique de contrôle de surface (25) sont représentés graphiquement les uns par rapport aux autres et/ou par rapport à l'os maxillaire (5) sur le cliché volumique (1) initial au moyen d'un dispositif d'affichage (17).

11. Procédé selon l'une des revendications 1 à 10, **caractérisé en ce que** les surfaces (13, 14, 15) des moignons dentaires (6, 7, 8) enregistrés du cliché volumique (1) initial en trois dimensions et/ou du cliché optique initial de surface (16) sont enregistrées dans une mémoire de données et les clichés optiques de contrôle de surface (25) sont explorés automatiquement au moyen d'un algorithme de reconnaissance de formes pour y trouver ces surfaces (13, 14, 15) enregistrées des moignons dentaires (6, 7, 8), la position et l'orientation des moignons dentaires (6, 7, 8) les uns par rapport aux autres et/ou par rapport à l'os maxillaire (5) étant ensuite déterminées de manière automatique assistée par ordinateur.
